# DEMANDE DE BREVET EUROPEEN

(11) **EP 4 011 455 A1**
(43) Date de publication de la demande: **15.06.2022**
(21) Numéro de dépôt: 21306718.4
(22) Date de dépôt: 07.12.2021
(51) Int. Cl.: A61Q 1/10, A61K 8/06, A61K 8/73

(54) **COMPOSITION COSMÉTIQUE COMPRENANT UN AMIDON HYDROXYALKYLÉ À TITRE DE SYSTÈME ÉMULSIONNANT PRINCIPAL**

(30) Priorité: 08.12.2020 FR 2012829
(71) Demandeur: Chanel Parfums Beauté, 92200 Neuilly-sur-Seine (FR)
(72) Inventeur: DE CLERMONT GALLERANDE, Hélène, 93645 PANTIN CEDEX (FR); COROLLER, Servane, 93645 PANTIN CEDEX (FR)
(74) Mandataire: Plasseraud IP

(57) **Abrégé**

La présente invention a pour objet une composition cosmétique notamment de revêtement des fibres kératiniques, se présentant sous forme d'émulsion cire-dans-eau et comprenant au moins un amidon hydroxyalkylé, caractérisée en ce que l'amidon hydroxyalkylé constitue le système émulsionnant principal de la composition.

Selon un autre de ses aspects, l'invention concerne un procédé de maquillage et/ou ou de soin non thérapeutique notamment des fibres kératiniques, comprenant l'application sur lesdites fibres kératiniques d'une composition telle que décrite précédemment.

La présente invention vise également l'utilisation d'une composition telle que décrite précédemment pour obtenir sur les fibres kératiniques, en particulier les cils, un maquillage homogène et/ou chargeant des cils.

## Description

### Domaine technique

La présente divulgation relève du domaine de la formulation cosmétique, et notamment de la formulation de mascaras.

### Technique antérieure

Parmi les formulations de mascaras, on connaît en particulier les mascaras dits « mascaras émulsions » ou « mascara washable », constitués d'une cire ou mélange de cires dispersées à l'aide d'au moins un tensioactif dans une phase aqueuse, contenant par ailleurs des polymères hydrosolubles ainsi que des pigments.

Ces particules solides, et en particulier les cires, sont généralement dispersées à l'aide d'un système tensioactif. Le choix du système tensioactif est prépondérant dans l'obtention d'une dispersion stable, dans la mesure où les tensioactifs jouent un rôle important à l'interface dans les interactions entre particules de cire au sein de la formule.

Le système tensioactif traditionnellement utilisé est composé de stéarate de triéthanolamine ou de ses dérivés.

En effet, ce système tensioactif permet de préserver la souplesse de la pâte formée, d'apporter un bon dépôt sur les cils et un volume persistant tout au long de la journée. Les stéarates de triéthanolamine et dérivés sont d'excellents tensioactifs pour les compositions mascaras, et sont donc difficilement substituables.

Cependant, les alcanoamines primaires, secondaires et tertiaires dont font partie les stéarates de triéthanolamine et dérivés sont responsables de la formation de nitrosamines. Ces nitrosamines sont des impuretés totalement indésirables dans les compositions cosmétiques de par leur toxicité.

La substitution des stératates de triéthanolamine et dérivés est donc une préoccupation importante des formulateurs dans le domaine cosmétique de nos jours.

Il existe des compositions présentant des systèmes tensioactifs à base de sucro esters en substitution des stéarates de triéthanolamine et dérivés. Cependant, les sucro esters ont l'inconvénient de ne pas apporter de volume sur les cils et seuls des résultats maquillage très discrets sont atteints par ce type de formulation. Un mascara volumateur n'est donc pas envisageable avec ce type de système émulsionnant.

Par conséquent, il subsiste le besoin de fournir des compositions cosmétiques de revêtement des fibres kératiniques, notamment des cils, exempts de triéthanolamine ou de ses dérivés, et possédant un pouvoir chargeant satisfaisant, permettant notamment de réaliser un maquillage épais des fibres kératiniques, en particulier des cils, dit encore maquillage chargeant.

### Résumé

La présente invention a précisément pour objet de répondre à ce besoin.

De façon surprenante et inattendue, les inventeurs de la présente demande ont résolu ce problème au moyen d'une composition cosmétique, notamment de revêtement des fibres kératiniques, plus particulièrement des cils, se présentant sous forme d'émulsion cire-dans-eau et comprenant au moins un amidon hydroxyalkylé à titre de système émulsionnant principal.

Plus précisément, les inventeurs ont pu observer que le système émulsionnant à base d'au moins un amidon hydroxyalkylé défini dans la présente demande permet d'obtenir une dispersion stable d'une quantité importante de cires, comparable à la dispersion obtenue avec un système émulsionnant à base de stéarate de triéthanolamine.

Au sens de la présente invention, le terme « fibres kératiniques » couvre les cheveux, les cils, les sourcils, et s'étend également aux postiches et faux-cils synthétiques.

Une « émulsion cire-dans-eau » entend désigner, au sens de la présente invention, une composition comprenant au moins une cire ou mélange de cires dispersée(s) à l'aide d'au moins un tensioactif dans une phase aqueuse continue.

Par «stable», on entend au sens de la présente invention, une composition qui, après avoir été placée dans une étuve à 20°C pendant deux mois, ne présente pas de grains perceptibles au toucher lorsqu'une couche fine de la composition est cisaillée entre les doigts.

Ainsi, l'invention concerne, selon un de ses aspects, une composition cosmétique de revêtement des fibres kératiniques sous la forme d'une émulsion cire-dans-eau comprenant au moins un premier système émulsionnant, appelé système émulsionnant principal, optionnellement un second système émulsionnant, appelé système émulsionnant secondaire, caractérisé en ce que le dit système émulsionnant principal consiste en un amidon hydroxyalkylé.

Avantageusement, une composition conforme à l'invention peut comprendre moins de 1%, de préférence moins de 0,5% en poids de triéthanolamine ou de ses dérivés, et mieux être exempte de triéthanolamine ou de ses dérivés. Cet élément ne constitue ni le système émulsionnant principal, ni le système émulsionnant secondaire.

De manière particulièrement avantageuse, une composition de l'invention peut comprendre une teneur en cires allant de 0,1% à 50%, préférentiellement de 1 à 40%, plus préférentiellement de 5 à 30% en poids, par rapport au poids total de la composition.

Par la teneur élevée de cires qu'elle peut incorporer, une composition selon l'invention présente ainsi une texture suffisamment épaisse pour obtenir un dépôt chargeant, volumateur sur les cils.

Les compositions de l'invention sont particulièrement adaptées au maquillage des cils, favorisant en particulier une application facile sur les cils ainsi que l'obtention d'un dépôt lisse et homogène.

Selon un autre de ses aspects, l'invention concerne un procédé de maquillage et/ou ou de soin non thérapeutique des fibres kératiniques, notamment des cils, comprenant l'application sur lesdites fibres kératiniques d'une composition telle que décrite précédemment.

La présente invention vise également l'utilisation d'une composition telle que décrite précédemment pour obtenir sur les fibres kératiniques, en particulier les cils, un maquillage homogène et/ou chargeant des cils.

Enfin, l'invention a pour objet l'utilisation de phosphate d'amidon hydroxypropylé comme unique système émulsionnant d'une composition cosmétique de mascara.

Au sens de la présente invention, on entend qualifier par le terme « chargeant » un maquillage épais des cils.

D'autres caractéristiques, propriétés et avantages de la présente invention apparaîtront plus clairement à la lecture de la description et des exemples qui suivent.

### Système émulsionnant principal

L'amidon hydroxyalkylé constitue le système émulsionnant principal de la composition.

Par « système émulsionnant principal », on entend un système qui permet à lui seul de stabiliser une emulsion. Ainsi, en son absence, la stabilité de l'émulsion ne peut pas être obtenue.

Avantageusement, l'amidon hydroxyalkylé constitue l'unique système émulsionnant de la composition.

En outre, les inventeurs ont remarqué que l'utilisation d'amidon hydroxyalkylé comme système tensioactif principal permet de diminuer fortement le pourcentage de gélifiant mis en œuvre dans la formule. En effet, l'amidon hydroxyalkylé gélifie la composition, lui conférant à la fois un rôle d'émulsionnant mais également de gélifiant. Son utilisation permet ainsi de limiter le nombre d'ingrédient utilisé pour la formulation d'un mascara.

Par « unique » on désigne une absence totale de tout autre système tensioactif. Ainsi, une composition qui présente comme unique système émulsionnant, un amidon hydroxyalkylé, présente donc un seul système émulsionnant dit principal et ne comprend pas de système émulsionnant secondaire ni de triéthanolamine ou dérivés dans sa composition.

L'amidon hydroxyalkylé selon l'invention peut être choisi parmi l'amidon hydroxyéthylé, l'amidon hydroxypropylé, et en particulier le phosphate d'amidon hydroxypropylé et le phosphate d'amidon hydroxyéthylé ou un mélange de ces composés.

Avantageusement, l'amidon hydroxyalkyl est le phosphate d'amidon hydroxypropylé. Cet amidon est notamment commercialisé par Akzo Nobel sous les références Structure@ ZEA et Structure@ XL.

L'amidon hydroxylalkylé peut être présent dans la composition en une teneur allant de 2 à 10% en poids, de préférence de 4 à 8% en poids et mieux autour de 5% en poids par rapport au poids total de la composition.

En effet, les meilleurs compromis entre stabilité et texture de la formule ont été obtenus dans ces teneurs en amidon hydroxylalkylé. Une teneur trop faible en amidon pourrait altérer la stabilité de la composition, alors qu'une teneur trop élevée pourrait générer trop de collant à la composition.

### Système émulsionnant secondaire

La composition cosmétique de revêtement des fibres kératiniques peut comprendre en plus du système émulsionnant principal, un système émulsionnant secondaire.

Par « système émulsionnant secondaire », on entend un système qui, vient compléter le système émulsionnant principal, mais ne suffit pas à lui seul pour obtenir une émulsion stable, de par sa nature ou sa quantité.

Le système émulsionnant secondaire peut être composé de tous émulsionnants connus dans l'état de l'art, à l'exception de la triéthanolamine ou un de ses dérivés. Les émulsionnants utilisés dans le système émulsionnant secondaire peuvent être des émulsionnants non ioniques, anioniques, cationiques ou amphotères. On peut se référer au document « Encyclopedia of Chemical Technology, KIRK-OTHMER », volume 22, 3ème édition, 1979, WILEY.

La composition selon l'invention comprend bien entendu un milieu physiologiquement acceptable.

Par « composé ou milieu physiologiquement acceptable » au sens de la présente demande, on entend un composé ou milieu dont l'utilisation est compatible avec une application sur les cils.

### Phase aqueuse

La composition selon l'invention comprend une phase aqueuse, qui forme la phase continue de la composition.

Par composition à phase continue aqueuse, on entend que la composition présente une conductivité, mesurée à 25°C, supérieure ou égale à 23 µS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les deux électrodes de la cellule. La lecture de la conductivité est faite dès que la valeur du conductimètre est stabilisée. Une moyenne est réalisée sur au moins 3 mesures successives.

La phase aqueuse comprend de l'eau et/ou au moins un solvant hydrosoluble. Par « solvant hydrosoluble », on désigne dans la présente invention un composé liquide à température ambiante et miscible à l'eau (miscibilité dans l'eau supérieure à 50% en poids à 25 °C et pression atmosphérique). Les solvants hydrosolubles utilisables dans les compositions selon l'invention peuvent en outre être volatils. Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en C 3 -C 4 et les aldéhydes en C2-C4.

La phase aqueuse (eau et éventuellement le solvant miscible à l'eau) est généralement présente dans la composition selon la présente demande en une teneur allant de 1% à 95% en poids, par rapport au poids total de la composition, de préférence allant de 3% à 80% en poids, et préférentiellement allant de 5% à 60% en poids.

Dans la composition conforme à l'invention, la teneur totale en agents tensioactifs peut aller de 1 à 13% en poids par rapport au poids total de la composition, de préférence de 3 à 10% et mieux autour de 5% en poids.

Selon un mode de réalisation, la composition cosmétique selon la présente demande comprend moins de 1%, de préférence moins de 0,5% en poids de triéthanolamine, et mieux, est exempte de triéthanolamine.

Selon une variante, la composition selon la présente demande comprend moins de 1%, de préférence moins de 0,5% en poids de stéarate de triéthanolamine, et mieux, est exempte de stéarate de triéthanolamine.

### Cire(s)

La composition selon la présente demande comprend au moins une cire.

Par cire au sens de la présente invention, on entend un composé lipophile, solide à température ambiante (25°C), à changement d'état solide/liquide réversible, ayant un point de fusion supérieur ou égal à 30°C pouvant aller jusqu'à 120°C. Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C.

La cire peut être présente en une teneur allant de 0,1 à 50% en poids par rapport au poids total de la composition, mieux de 1 à 40% et encore mieux de 5 à 30% en poids. On peut notamment utiliser les cires hydrocarbonées comme la cire d'abeille, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50^{®}, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination HEST 2T-4S" par la société HETERENE, le tétrabéhénate de di-(triméthylol-1,1,1 propane) vendu sous la dénomination HEST 2T-4B par la société HETERENE. On peut encore citer les cires de silicone comme les alkyl ou alkoxy-diméticone ayant de 16 à 45 atomes de carbone, les cires fluorées.

On peut également utiliser la cire obtenue par hydrogénation d'huile d'olive estérifiée avec l'alcool stéarylique vendue sous la dénomination "PHYTOWAX Olive 18 L 57" ou bien encore les cires obtenues par hydrogénation d'huile de ricin estérifiée avec l'alcool cétylique vendus sous la dénomination "PHYTOWAX ricin 16L64 et 22L73", par la société SOPHIM. De telles cires sont décrites dans la demande FR-A- 2792190.

Selon un mode de réalisation particulier, les compositions conformes à l'invention peuvent comprendre au moins une cire dite cire collante c'est-à-dire possédant un collant supérieur ou égal à 0,7 N.s et une dureté inférieure ou égale à 3,5 MPa. L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 .deg.C à l'aide du texturomètre vendu sous la dénomination "TA-TX2i(R)" par la société RHEO, équipé d'un mobile en polymère acrylique en forme de cône formant un angle de 45 degrés. Le protocole de mesure est le suivant :
- La cire est fondue à une température égale au point de fusion de la cire + 10°C. La cire fondue est coulée dans un récipient de 25 mm de diamètre et de 20 mm de profondeur.
- La cire est recristallisée à température ambiante (25°C) pendant 24 heures de telle sorte que la surface de la cire soit plane et lisse, puis la cire est conservée pendant au moins 1 heure à 20°C avant d'effectuer la mesure du collant.
- Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.
- Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en C20- C40 le groupe alkyle comprenant de 20 à 40 atomes de carbone), seul ou en mélange, en particulier un 12-(12'-hydroxystéaryloxy)stéarate d'alkyle en C20-C40.

Une telle cire est notamment vendue sous les dénominations "Kester Wax K 82 P^{®}" et 5 "Kester Wax K 80 P^{®}" par la société KOSTER KEUNEN.

Les cires citées ci-dessus présentent généralement un point de fusion commençante inférieur à 45°C.

On peut également utiliser la cire microcristalline commercialisée sous la référence SP18 par la société STRAHL and PITSCH qui présente une dureté d'environ 0,46 MPa 10 et une valeur de collant d'environ 1 N.s.

La ou les cires peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 pm.

Les microdispersions de cire sont des dispersions stables de particules colloïdales de cire, et sont notamment décrites dans "Microemulsions Theory and Practice", L.M. Prince Ed. Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 pm (notamment allant de 0,02 pm à 0,99 pm), de préférence inférieures à 0,5 pm (notamment allant de 0,06 pm à 0,5 pm).

Ces particules sont constituées essentiellement d'une cire ou d'un mélange de cires.

Elles peuvent toutefois comprendre en proportion minoritaire des additifs gras huileux et/ou pâteux, un tensioactif et/ou un additif/actif liposoluble usuel.

### Polymère filmogène

Les compositions selon la présente demande peuvent aussi contenir au moins un polymère filmogène hydrophile ou lipophile.

Dans la présente demande, on entend par "polymère filmogène", un polymère apte à former à lui seul ou en présence d'un agent auxiliaire de filmification, un film macroscopiquement continu et adhérent sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface anti-adhérente comme une surface téflonée ou siliconée.

De façon générale, la teneur en "polymère filmogène" des compositions selon la présente demande va de 0,1 à 40% de préférence de 0,5 à 30% mieux de 1 à 20% en poids, par rapport au poids total de la composition.

Le polymère filmogène hydrophile peut être un polymère hydrosoluble ou se présenter en dispersion dans un milieu aqueux.

Parmi les polymères filmogènes utilisables dans la composition de la présente invention, on peut citer les polymères synthétiques, de type radicalaire ou de type polycondensat, les polymères d'origine naturelle, et leurs mélanges.

Comme exemples de polymères filmogènes hydrosolubles, on peut citer :
- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères de chitine ou de chitosane anioniques, cationiques, amphotères ou non-ioniques ;
- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléique;
- les muccopolysaccharides tels les chondroïtines sulfate, et leurs mélanges ;
- les polysaccharides naturels tels que ceux produits à partir d'amidon par fermentation utilisant une levure naturelle tels que le pullulan^{®} commercialisé par la société Hayashibara international, ou le galactoarabinan commercialisé sous le nom de Laracare A200^{®} par la société Larex.
- les mélanges d'oligosaccharides tels que l'inuline vendue sous la référence Inutec H25P^{®} par la société Naturochim.

Le polymère filmogène peut être également présent dans la composition sous la forme de particules en dispersion dans une phase aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90 ^{®} , Neocryl A-1070 ^{®} , Neocryl A-1090 ^{®}, Neocryl BT-62 ^{®}, Neocryl A-1079 ^{®} et Neocryl A-523 ^{®} par la société AVECIA-NEORESINS, Dow Latex 432 ^{®} par la société DOW CHEMICAL, Daitosol 5000 AD ^{®} ou Daitosol 5000 SJ ^{®} par la société DAITO KASEY KOGYO; Syntran 5760 ^{®} par la société Interpolymer Allianz Opt ^{®} par la société Rohm and Haas, ou encore les dispersions aqueuses de polyuréthane vendues sous les dénominations Neorez R-981 ^{®} et Neorez R-974 ^{®} par la société AVECIA-NEORESINS, les Avalure UR-405 ^{®} , Avalure UR-410 ^{®} , Avalure UR-425^{®}, Avalure UR-450^{®}, Sancure 875^{®}, Avalure UR-445 ^{®} et Sancure 2060 ^{®} par la société NOVEON, Impranil 85 ^{®}, BaycusanC1001^{®}, BaycusanC1004^{®} par la société BAYER, Aquamere H-1511 ^{®} par la société HYDROMER ; les sulfopolyesters vendus sous le nom de marque Eastman AQ ^{®} par la société Eastman Chemical Products, les dispersions vinyliques comme le Mexomère PAM ^{®} , les dispersions aqueuses de polyvinyl acétate comme le "Vinybran ^{®} " de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références "Hybridur ^{®} " par la société AIR PRODUCTS ou "Duromer ^{®} " de NATIONAL STARCH, les dispersions type core/shell : par exemple celles commercialisées par la société ATOFINA sous la référence Kynar (core : fluoré shell :
acrylique) ou encore ceux décrits dans le document US 5 188 899 (core ; silice - shell : silicone) et leurs mélanges.

Le polymère lipophile peut être en solution ou en dispersion dans une phase solvant non aqueuse ou bien dispersé dans un mélange de cires. Le polymère lipophile peut être en particulier un copolymère éthylène/ acétate de vinyle.

Il est généralement présent en quantité suffisante dans la composition pour éviter la formation de paquets lors de l'application de la formule sur les cils.

La quantité de copolymère éthylène / acétate de vinyle pourra ainsi être ajustée en fonction du taux de cires présent dans la composition de l'invention.

Le rapport pondéral du copolymère éthylène/ acétate de vinyle à la cire ou au mélange de cires dans la composition de l'invention est de préférence compris entre 1 :55 et 1 :9.

De préférence le rapport pondéral du copolymère éthylène/ acétate de vinyle à la cire ou au mélange de cires dans la composition de l'invention est compris entre 1 :55 et 1 :15.

De manière encore plus préférentielle, le rapport pondéral du copolymère éthylène/ acétate de vinyle à la cire ou au mélange de cires dans la composition de l'invention est compris entre 1 :50 et 1 :20.

Dans un mode de réalisation préféré de l'invention, on utilise un copolymère éthylène/acétate de vinyle préalablement dispersé dans un mélange de cires.

Cela présente l'avantage de diminuer le point de fusion du copolymère, ce qui permet de diminuer la température de chauffe pour la préparation de la composition de l'invention.

Les cires servant à la prédispersion du copolymère de l'invention peuvent être choisies parmi celles qui sont mentionnées ci-après. De préférence, les cires utilisées pour prédisperser le polymère sont choisies parmi la cire synthétique, la cire microcristalline ou leur mélange.

En particulier, on peut utiliser un pré-mélange de copolymère dans des cires comprenant 80% en poids de copolymère éthylène/ acétate de vinyle, dans lequel le pourcentage d'acétate de vinyle est de 13% en poids par rapport au poids total du copolymère, le copolymère étant pré-dispersé dans un mélange de cires comprenant 15% en poids de cire microcristalline et 5% en poids de cire synthétique commercialisé sous la dénomination Cérylène B72^{®} par la société Baerlocher.

### Gélifiants

Les compositions selon la présente demande peuvent aussi contenir au moins un composé gélifiant, de préférence hydrophile, pouvant être choisi parmi :
- les homo- ou copolymères d'acides acrylique ou méthacrylique ou leurs sels et leurs esters et en particulier les produits vendus sous les dénominations VERSICOL F ^{®} ou VERSICOL K ^{®} par la société ALLIED COLLOID, UTRAHOLD 8 ^{®} par la société CI BA-GEIGY, les acides polyacryliques de type SYNTHALEN K ;
- les copolymères d'acide acrylique et d'acrylamide vendus sous la forme de leur sel de sodium sous les dénominations RETEN ^{®} par la société HERCULES, les sels de sodium d'acides polyhydroxycarboxyliques vendus sous la dénomination HYDAGEN F ^{®} par la société HENKEL ;
- les copolymères acide polyacryliques/acrylates d'alkyle de type PEMULEN ;
- l'AMPS (Acide polyacrylamidométhyl propane sulfonique neutralisé partiellement à l'ammoniaque et hautement réticulé) commercialisé par la société CLARIANT ;
- les copolymères AMPS/acrylamide de type SEPIGEL ^{®} ou SIMULGEL ^{®} commercialisés par la société SEPPIC ;
- les copolymères AMPS/méthacrylates d'alkyle polyoxyéthylénés (réticulés ou non), et leurs mélanges.
- les polyuréthanes associatifs tels que le polymère C 16 -0E 120 -C 16 de la société SERVO DELDEN (commercialisé sous le nom SER AD FX1100, molécule à fonction uréthanne et poids moléculaire moyen en poids de 1300), OE étant un motif oxyéthyléné, le Rhéolate 205 à fonction urée vendu par la société RHEOX ou encore le Rhéolate 208 ou 204 (ces polymères étant vendus sous forme pure) ou le DW 1206B de chez RHOM & HAAS à chaîne alkyle en C20 et à liaison uréthane, vendu à 20% en matière active dans l'eau.

On peut aussi utiliser des solutions ou dispersions de ces polyuréthanes associatifs notamment dans l'eau ou en milieu hydroalcoolique. A titre d'exemple, de tels polymères on peut citer, le SER AD fx1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCH ERS, et leurs mélanges.

Certains polymères filmogènes hydrosolubles cités plus haut peuvent également jouer le rôle de gélifiant hydrosoluble.

Selon un mode particulier de réalisation, la composition selon l'invention comprend moins de 1%, de préférence moins de 0,5% en poids de composés gélifiants, et mieux, est exempte de composé gélifiant. En effet, l'amidon hydroxyalkylé mis en œuvre dans la présente demande ayant des propriétés gélifiantes, il n'est généralement pas nécessaire d'ajouter d'autres gélifiants pour garantir l'obtention d'une émulsion stable, présentant la texture recherchée.

Il est toutefois possible d'ajouter des gélifiants selon la texture souhaitée pour la composition selon l'invention. S'ils sont présents dans la composition, les gélifiants hydrophiles peuvent être introduits en une teneur allant de 0,05 à 40% en poids, par rapport au poids total de la composition, de préférence de 0,1 à 20% et mieux de 0,5 à 15% en poids.

### Huiles

Les compositions selon la présente demande peuvent aussi contenir au moins une ou plusieurs huiles ou solvant organique.

Par huile ou solvant organique, on entend un corps non aqueux liquide à température ambiante et pression atmosphérique. L'huile peut être volatile ou non volatile.

Par " huile ou solvant organique volatile", on entend au sens de l'invention tout milieu non aqueux susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant de 0,13 Pa à 40 000 Pa (10 3 à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par "huile non volatile", on entend une huile restant sur les matières kératiniques à température ambiante et pression atmosphérique au moins plusieurs heures et ayant notamment une pression de vapeur inférieure à 10 -3 mm de Hg (0,13Pa).

L'huile peut être présente dans la composition dans une teneur allant de 0,05 à 30% de préférence 0,1 à 15% en poids par rapport au poids total de la composition. La composition selon l'invention peut comprendre des huiles volatiles et/ou des huiles non volatiles, et leurs mélanges.

Les huiles (ou solvants organiques) volatiles peuvent être des huiles hydrocarbonées, des huiles siliconées, des huiles fluorées ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C 8 -C 16 comme les isoalcanes en C 8 -C 16 d'origine pétrolière (appelées aussi isoparaffines) comme l'isododécane (encore appelé 2,2,4,4,6pentaméthylheptane), l'isodécane, l'isohexadécane, et par exemple les huiles vendues sous les noms commerciaux « d'isopars ^{®} » ou de « Permetyls^{®} » les esters ramifiés en C8- C16, le néopentanoate d'iso-hexyle, et leurs mélanges.

D'autres huiles hydrocarbonées volatiles comme les distillats de pétrole, notamment ceux vendus sous la dénomination "Shell Soit ^{®} " par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité 6 centistokes (6.10 -6 m 2 /s), et ayant notamment de 3 à 6 atomes de silicium, ces silicones comportant éventuellement un ou plusieurs groupes alkyles ou alkoxy ayant de 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants organiques volatils notamment fluorés tels que le nonafluorométhoxy butane ou le perfluorométhylcyclopentane.

Chacune des compositions conformes à l'invention peut également comprendre au moins une huile ou solvant organique non volatile, qui peut être en particulier choisie parmi les huiles hydrocarbonées et/ou siliconées et/ou fluorées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C4 à C24, ces derniers pouvant être linéaires ou ramifiés, saturés ou insaturés ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations de Miglyol 810 ^{®} , 812 ^{®} et 818 ^{®} par la société Dynamit Nobel ;
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1 COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthylhexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique; et leurs mélanges.

Les huiles de silicone non volatiles utilisables dans l'une ou l'autre des compositions (i) ou (ii) conformes à l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les huiles fluorées utilisables dans les compositions conformes à l'invention sont notamment des huiles fluorosiliconées, des polyéthers fluorés, des silicones fluorées telles que décrit dans le document EP-A-847752.

La teneur en huile ou solvant organique non volatile dans la composition conforme à l'invention va de 0,01 à 30% en poids, en particulier de 0,1 à 25% en poids, et mieux de 0,1 à 20% par rapport au poids total de la composition.

### Matière colorante

Les compositions conformes à l'invention peuvent également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6 , le 13-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30% en poids par rapport au poids total de la composition.

### Charges

Les compositions conformes à l'invention peuvent également comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon ^{®}commercialisé sous la dénomination Orgasol ^{®} par la société Atochem, de poly-13-alanine et de polyéthylène, les poudres de polymères de tétrafluoroéthylène comme le Téflon ^{®} , la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel ^{®} par la société Nobel Industrie, les poudres acryliques telles que celles commercialisées sous la dénomination Polytrap ^{®} par la société Dow Corning, les particules de polyméthacrylate de méthyle et les microbilles de résine de silicone (Tospearls ^{®} de Toshiba, par exemple), le carbonate de calcium précipité, le carbonate et l'hydrocarbonate de magnésium, l'hydroxyapatite, les microsphères de silice creuses (Silica Beads ^{®} de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

On peut également utiliser un composé susceptible de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel ^{®} 820 DU 40 et Expancel ^{®} 007WU par la Société AKZO NOBEL. On peut citer également les Advancell^{®} commercialisées par Sekuisi Plastic ou les Thermoexpandable microspheres^{®} de Matsumoto.

Les charges peuvent représenter de 0,1 à 25% en particulier de 0,2 à 20% en poids par rapport au poids total de la composition.

### Fibres

Les compositions conformes à l'invention peuvent également comprendre au moins une fibre qui permet une amélioration de l'effet allongeant.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur section peut être quelconque et notamment de section circulaire ou polygonale (carrée, hexagonale ou octogonale) selon l'application spécifique envisagée. Leur forme peut être linéaire, courbe, sinusoïdale ou frisée. En particulier, leurs extrémités sont épointées et/ou polies pour éviter de se blesser.

En particulier, les fibres ont une longueur allant de 1 µm à 10 mm, en particulier de 0,1 mm à 5 mm et plus particulièrement de 0,3 mm à 3,5 mm.

Leur section peut être comprise dans un cercle de diamètre allant de 2 nm à 500 µm, en particulier allant de 100 nm à 100 µm et plus particulièrement de 1 µm à 50 µm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origines synthétiques ou naturelles, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon^{®}) ou les fibres rigides telles que les fibres de polyimide-amide comme celles vendues sous les dénomination KERMEL^{®}, KERMEL TECH^{®} par la société RHODIA ou de poly-(p-phénylènetéréphtalamide) (ou d'aramide) notamment vendues sous la dénomination Kevlar^{®} par la société DUPONT DE NEMOURS.

Les fibres peuvent être présentes dans la composition selon l'invention en une teneur allant de 0,01% à 10% en poids, par rapport au poids total de la composition, en particulier de 0,1% à 5% en poids, et plus particulièrement de 0,3% à 3% en poids. Les compositions conformes à l'invention peuvent également comprendre au moins un actif cosmétique.

### Actifs cosmétiques

Comme actifs cosmétiques pouvant être utilisés dans les compositions conformes à l'invention, on peut citer notamment les antioxydants, les conservateurs, les parfums, les neutralisants, les émollients, les épaississants, les agents de coalescence, les plastifiants, les hydratants, les vitamines et les filtres en particulier solaires, et leurs mélanges.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

En modifiant la quantité des cires, des gélifiants, des polymères filmogènes et des charges, il est possible d'obtenir un résultat maquillage satisfaisant du point de vue de l'allongement, du recourbement et du volume des cils.

### Exemples

Les compositions suivantes ont été réalisées. Les quantités indiquées sont exprimées en pourcentage massique par rapport au poids total de la composition.

Le [Tableau 1] exemplifie une formule de mascara à base de phosphate d'amidon hydroxypropylé sous la dénomination commerciale Structure@ XL de Akzo Nobel.

Cet émulsionnant est utilisé dans l'exemple A ci-dessous à 4% et correspond à l'invention. L'exemple B correspond à la formule comparative avec utilisation de triéthanolamine pour assurer la stabilité de l'émulsion.

**[Tableau 1]**

| Nom INCl | Exemple A (%) | Exemple B (%) |
|---|---|---|
| Hydroxypropyl starch phosphate & water | 4,00 | - |
| Triéthanolamine | - | 1,67 |
| Stearic acid & palmitic acid | - | 3,00 |
| C 10-18 Triglycérides | 4,00 | 4,00 |
| Glyceryl béhénate | 5,00 | 5,00 |
| Beeswax & benzyl alcohol & benzyl cinnamate & benzyl benzoate | 5,00 | 5,00 |
| Glyceryl hydroxystearate | 2,50 | 2,50 |
| Jojoba esters & polyglycerin-3 & acacia decurrens flower wax & helianthus annuus seed wax | 4,50 | 4,50 |
| Octyldodecyl myristate | 2,00 | 2,00 |
| Sucrose acetate isobutyrate | 1,00 | 1,00 |
| Castor seed oil & hydrogenated castor oil | 2,00 | 2,00 |
| Water | 48,65 | 48,83 |
| Phenylpropanol & propanediol & caprylyl glycol & tocophérol | 1,00 | 1,00 |
| Pullulan & sorbitol & acacia senegal gum & tréhalose | 5,00 | 5,00 |
| Glycerin | 5,00 | 5,00 |
| Tocopheryl acetate | 0,30 | 0,30 |
| Pigments Iron Oxides | 10 | 10 |
| TOTAL | 100,00 | 100,00 |

La formule de mascara B est une formule classique, connue de l'état de l'art, servant de témoin. Celle-ci présente une consistance satisfaisante (en particulier par l'ajout de « steraic acid & palmitic acid » qui joue un role d'épaississant et de co-émulsionnant dans l'exemple B, permettant d'obtenir une composition B la plus proche possible de l'exemple A en termes de texture) et une bonne dispersion des cires et pigments qui la composent. On obtient ainsi un produit de couleur noir, homogène qui s'applique facilement sur les cils et forment un dépôt chargeant, lisse et un effet volume souhaité.

L'invention permet d'obtenir un même résultat mais sans utiliser de stéarates de triéthanolamine ou dérivés pour former l'émulsion de la composition.

La formule mascara de l'exemple A présente une consistance satisfaisante et une bonne dispersion des cires et pigments qui assure une teinte noire, telle que recherchée pour ce type de produit.

Ces mascaras s'appliquent facilement sur les cils et forment un dépôt chargeant, lisse et homogène et procurent un résultat volumateur recherché.

On obtient alors un mascara en émulsion cire-dans-eau stable, exempt de triéthanolamine ou dérivés et présentant un résultat maquillage tout à fait satisfaisant et comparable à un mascara avec triéthanolamine ou dérivés.

On note à l'issu de ces différents tests, que le Structure@ XL permet non seulement d'assurer la stabilité d'une émulsion cire-dans-eau mais également de jouer sur la texture de la formule et d'obtenir des émulsions plus ou moins élastiques, plus ou moins liquide, ce qui représente un grand atout pour le formulateur.

A l'inverse d'une formule émulsionnée avec des stéarates de triéthanolamine qui nécessite une quantité non négligeable d'agents de texture supplémentaires pour obtenir une formule de consistance suffisante, une formule émulsionnée avec un amidon hydroxylé ne nécessite pas ou peu d'agents de texture.

Le résultat maquillage d'une composition cosmétique pour les cils contenant le Structure^{®} XL est le suivant : les cils sont gainés sur toute la longueur.

## Revendications

1. Composition cosmétique de revêtement des fibres kératiniques sous la forme d'une émulsion cire-dans-eau comprenant un système émulsionnant, **caractérisée en ce que** le dit système émulsionnant consiste en un amidon hydroxyalkylé et constitue l'unique système émulsionnant de la composition.

2. Composition selon la revendication précédente, **caractérisée en ce que** l'amidon hydroxyalkylé est choisi parmi l'amidon hydroxyéthylé, l'amidon hydroxypropylé,et en particulier le phosphate d'amidon hydroxypropylé et le phosphate d'amidon hydroxyéthylé.

3. Composition selon l'une des revendications précédentes, **caractérisée en ce que** l'amidon hydroxyalkylé est le phosphate d'amidon hydroxypropylé.

4. Composition selon l'une des revendications précédentes, **caractérisée en ce que** l'amidon hydroxyalkylé est présent en une teneur allant de 2 à 10% en poids, de préférence de 4 à 8% en poids et mieux autour de 5% en poids par rapport au poids total de la composition.

5. Composition selon l'une des revendications précédentes, **caractérisée en ce qu'**elle comprend une phase aqueuse présente en une teneur allant de 1% à 95% en poids, par rapport au poids total de la composition, de préférence allant de 3% à 80% en poids, et préférentiellement allant de 5% à 60% en poids.

6. Composition selon l'une des revendications précédentes, **caractérisée en ce que** la cire est présente en une teneur allant de 0,1% à 50% en poids, par rapport au poids total de la composition, de préférence de 1% à 40% en poids, et préférentiellement allant de 5% à 30% en poids.

7. Composition selon l'une des revendications précédentes **caractérisée en ce qu'**elle comprend au moins un polymère filmogène hydrophile ou lipophile.

8. Composition selon l'un des revendications précédentes **caractérisée en ce qu'**elle comprend moins de 1%, de préférence moins de 0,5% en poids de composés gélifiants, et mieux, est exempte de composé gélifiant.

9. Composition selon l'une des revendications précédentes **caractérisée en ce qu'**elle comprend au moins un additif choisi parmi les huiles, les matières colorantes, les charges, les fibres, les antioxydants, les conservateurs, les parfums, les neutralisants, les émollients, les épaississants, les agents de coalescence, les plastifiants, les hydratants, les vitamines et les filtres en particulier les filtres solaires, et leurs mélanges.

10. Utilisation de phosphate d'amidon hydroxypropylé comme unique système émulsionnant d'une composition cosmétique de mascara.
